Europäisches Patentamt

European Patent Office

Office européen des brevets

(11) Veröffentlichungsnummer: **0 517 939 A1**

(12) **EUROPÄISCHE PATENTANMELDUNG**

(21) Anmeldenummer: **91109626.1**

(22) Anmeldetag: **12.06.91**

(51) Int. Cl.⁵: **A61B 17/60**

(43) Veröffentlichungstag der Anmeldung:
**16.12.92 Patentblatt 92/51**

(84) Benannte Vertragsstaaten:
**AT BE CH DE DK ES FR GB GR IT LI LU NL SE**

(71) Anmelder: **FEHLING MEDIPRODUKT GMBH**
**Frankenstrasse 21**
**W-8757 Karlstein(DE)**

(72) Erfinder: **Kretschmer, Peter**
**Konr.-Adenauer-Strasse 14**
**W-6454 Bruchköbel(DE)**
Erfinder: **Hessler, Winfried**
**Am Heiligen Wald 7**
**W-8752 Mömbris(DE)**
Erfinder: **Baumgart, Rainer, Dr.**
**Athener Platz 11**
**W-8000 München(DE)**
Erfinder: **Betz, Augustin, Dr.**
**Am Sonnengrund 4a**
**W-8130 Starnberg(DE)**

(74) Vertreter: **TER MEER - MÜLLER -**
**STEINMEISTER & PARTNER**
**Mauerkircherstrasse 45**
**W-8000 München 80(DE)**

(54) **Medizinische Fixierungsvorrichtung.**

(57) Eine medizinische Fixierungsvorrichtung für die Osteosynthese besteht aus einem langgestreckten Überbrückungsbauteil (1), mit welchem mit Knochen verbindbare stabförmige Fixierungselemente (2) über einen sie axial aufnehmenden Klemmring (3) verklemmbar sind, der in einer kegelstumpfartigen ersten Pfanne (10) des Überbrückungsbauteils (1) liegt und durch eine in das Überbrückungsbauteil (1) einschraubbare Klemmschraube (4), die eine kegelstumpfartige zweite Pfanne (12) zur Aufnahme des Klemmrings (3) aufweist, verformbar ist. Der Öffnungswinkel ($\alpha$) der kegelstumpfartigen ersten Pfanne (10) ist dabei kleiner als der Öffnungswinkel ($\beta$) der kegelstumpfartigen zweiten Pfanne (12), so daß beim Klemmvorgang zunächst der Klemmring (3) festgesetzt und damit ein Verdrehen oder eine axialen Verschiebung des Fixierungselements (2) beim weiteren Anziehen der Klemmschraube (4) verhindert wird.

Fig. 1

Die Erfindung betrifft eine medizinische Fixierungsvorrichtung gemäß dem Oberbegriff des Patentanspruchs 1.

Eine derartige medizinische Fixierungsvorrichtung ist bereits aus der EP 0 240 034 A1 bekannt. Sie besteht gemäß der dortigen Fig. 14 aus einem langgestreckten Überbrückungsbauteil, mit welchem mit Knochen verbindbare stabförmige Fixierungselemente über einen sie axial aufnehmenden Klemmring verklemmbar sind, der in einer kegelstumpfartigen ersten Pfanne des Überbrückungsbauteils liegt und durch eine in das Überbrückungsbauteil einschraubbare Klemmschraube, die eine kegelstumpfartige zweite Pfanne zur Aufnahme des Klemmrings aufweist, verformbar ist.

Diese Fixierungsvorrichtung weist neben einer großen Bauhöhe den Nachteil auf, daß sich zu einem relativ frühen Zeitpunkt beim Einschrauben der Klemmschraube der Klemmring innerhalb der zweiten Pfanne festsetzt, und zwar unter Anlegung an das Fixierungselement, was zu dessen unerwünschter Mitnahme führt.

Eine weitere Fixierungsvorrichtung für Röhrenknochenfrakturen ist aus der DE 39 12 703 C2 bekannt. Dort erfolgt die axiale Fixierung einer Knochenschraube mittels eines längsgeschlitzten Konus, der über die Knochenschraube geschoben wird und, angepreßt durch eine mit einem flachen Kopf versehene Schraube, die in der Mitte das abgeschnittene Ende der Knochenschraube aufnimmt, seinen festen Halt in einem ebenfalls konischen Gegenlager im Bereich einer kegelsegmentartigen Führung findet.

Die den flachen Kopf aufweisende Schraube ist allerdings nicht drehgesichert. Es besteht daher die Gefahr, daß sie sich wieder löst und die Verklemmung zwischen Fixierungselement und Überbrückungsbauteil aufgehoben wird, wenn die Fixierungsvorrichtung von einem Patienten über längere Zeit getragen wird und entsprechenden Erschütterungen ausgesetzt ist.

Der Erfindung liegt die Aufgabe zugrunde, eine medizinische Fixierungsvorrichtung der eingangs genannten Art zu schaffen, bei der zu jedem Zeitpunkt ein Mitdrehen der Fixierungselemente beim Einsetzen der Klemmschrauben und ein Lösen der Klemmschrauben beim Tragen der Fixierungsvorrichtung sicher verhindert werden.

Die Lösung der gestellten Aufgabe ist im kennzeichnenden Teil des Patentanspruchs 1 angegeben. Vorteilhafte Ausgestaltungen der Erfindung sind den Unteransprüchen zu entnehmen.

Die medizinische Fixierungsvorrichtung nach der Erfindung zeichnet sich dadurch aus, daß ein Öffnungswinkel der kegelstumpfartigen ersten Pfanne kleiner ist als ein Öffnungswinkel der kegelstumpfartigen zweiten Pfanne.

Hierdurch wird erreicht, daß beim Einschrauben der Klemmschraube in das Überbrückungsbauteil der Klemmring zuerst im Bereich der kegelstumpfartigen ersten Pfanne festgesetzt wird, wodurch schon frühzeitig eine Sicherung des Fixierungselements gegen Verdrehen und axiales Verschieben zustande kommt. Bei weiterem Festziehen der Klemmschraube wird die Klemmwirkung noch erhöht, wobei zusätzlich eine Verklemmung der Klemmschraube im Bereich ihrer kegelstumpfartigen zweiten Pfanne auftritt. Diese Verklemmung bewirkt eine Sicherung der Klemmschraube gegen eine Lockerung der Verschraubung insbesondere auch bei einer über längere Zeit auftretenden dynamischen bzw. periodischen Beanspruchung, so daß eine dauerhaft feste Verbindung zwischen den Fixierungselementen und dem Überbrückungsbauteil erhalten wird.

Nach einer vorteilhaften Ausgestaltung der Erfindung weist der Klemmring an seinen gegenüberliegenden axialen Enden jeweils einen konusförmigen Verlauf auf, der mit dem der jeweiligen ersten bzw. zweiten Pfanne übereinstimmt. Die konusförmigen Endflächen des Klemmrings liegen somit parallel zu und auf den jeweiligen kegelstumpfartigen Pfannenflächen, so daß eine flächige bzw. gleichmäßige Kraftübertragung zwischen Klemmschraube und Klemmring einerseits sowie zwischen Überbrückungsbauteil und Klemmring andererseits erhalten wird, um den Klemmring möglichst gleichmäßig und auch beschädigungsfrei verformen zu können.

Nach einer anderen vorteilhaften Ausgestaltung der Erfindung besitzt der Klemmring eine kugelförmige Außenfläche, zu der die Pfannenflächen tangential verlaufen. Der Klemmring ist praktisch eine Kugel mit zentraler Durchgangsöffnung für das Fixierungselement. Demzufolge berühren sich Klemmring und erste Pfanne bzw. zweite Pfanne entlang von Kreislinien. Ein derart ausgebildeter Klemmring kann vor Einklemmung zwischen erster und zweiter Pfanne axial verkippt werden, so daß eine gewisse Winkelausrichtung der Fixierungselemente relativ zum Überbrückungsbauteil möglich ist.

Die Klemmringe der genannten Art können z. B. aus plastisch oder elastisch verformbarem Material bestehen, so daß sie ein- oder mehrmals zur Verklemmung herangezogen werden können.

Sie können nach einer vorteilhaften Weiterbildung der Erfindung aber auch an einer Umfangsposition vollständig in Axialrichtung geschlitzt sein, um beim Einklemmvorgang radial verformt werden zu können.

Nach einer sehr vorteilhaften weiteren Ausgestaltung der Erfindung weist der Klemmring an seiner äußeren Umfangsfläche axial verlaufende Ausnehmungen auf, durch die sich der Klemmring besonders gleichmäßig in Umfangsrichtung gegen die äußere Fläche des Fixierungselements drücken läßt. Insbesondere erhält der Klemmring durch diese axial verlaufenden Ausnehmungen eine kleinere Federkonstante, so daß der Klemmvorgang mit weniger Kraftaufwand erfolgen kann. Darüber hinaus ist man bei Verwendung der axialen Ausnehmungen flexibler in der Wahl des Durchmessers der Fixierungselemente, da sich der Klemmring dann auch gegen dünnere Fixierungselemente pressen läßt.

Als besonders vorteilhaft erweisen sich die an der äußeren Umfangsfläche axial verlaufenden Ausnehmungen der Klemmringe dann, wenn die Klemmringe aus metallischen Materialien hergestellt sind, beispielsweise aus rostfreiem Stahl, aus Titan oder Titanlegierungen, aus Kobalt-Basis-Legierungen oder sonstigen Implantatwerkstoffen. Aus diesen genannten Materialien können auch die restlichen Bauteile der Fixierungsvorrichtung nach der Erfindung bestehen. Die Bauteile können aber auch aus anderen geeigneten Implantatwerkstoffen hergestellt sein, beispielsweise aus Kunststoffen, aus keramischen Werkstoffen, usw.

Es hat sich gezeigt, daß hinsichtlich der Verklemmung der Fixierungselemente und der Drehsicherung der Klemmschraube dann gute Ergebnisse erzielt werden, wenn der Öffnungswinkel der kegelstumpfartigen ersten Pfanne im Bereich von 10° bis 40° zu liegen kommt und der Öffnungswinkel der kegelstumpfartigen zweiten Pfanne im Bereich von 50° bis 100° liegt. Bei metallischen Werkstoffen haben sich ein Öffnungswinkel der ersten Pfanne von etwa 24° und ein Öffnungswinkel der zweiten Pfanne von etwa 60° als vorteilhaft erwiesen. Unter dem Öffnungswinkel ist jeweils derjenige Winkel zu verstehen, der in einer Axialebene der Schraubverbindung von Klemmschraube und Überbrückungsbauteil liegt und den Winkel beschreibt, unter dem die jeweils einander gegenüberliegenden Kegelflächen der ersten und der zweiten Pfanne verlaufen.

Um eine geringe Bauhöhe der medizinischen Fixierungsvorrichtung nach der Erfindung erhalten zu können, sind die Klemmschrauben vorzugsweise als Flachkopfschrauben oder als Schrauben ohne Kopf ausgebildet, die sich ganz oder fast vollständig in das Überbrückungsbauteil hineinschrauben lassen.

Die medizinische Fixierungsvorrichtung nach der Erfindung läßt sich, ohne daß hierdurch ihr Einsatzgebiet eingeschränkt werden soll, vorzugsweise zur operativen Stabilisierung von Röhrenknochenfrakturen im Arm- und Beinbereich verwenden, aber auch für Stabilisierungsaufgaben im Bereich der Wirbelsäule einsetzen.

Nach einer sehr vorteilhaften weiteren Ausgestaltung der Erfindung ist der Öffnungswinkel α der ersten Pfanne bis zu 4° größer als der Kegelwinkel des in ihr liegenden zweiten Konus des Klemmrings. Auch der Öffnungswinkel β der zweiten Pfanne ist vorzugsweise nach einer noch weiteren Ausgestaltung der Erfindung bis zu 4° größer als der Kegelwinkel des in ihr liegenden ersten Konus des Klemmrings.

Hierdurch wird sichergestellt, daß die Klemmung zunächst an den stirnseitigen Außenkanten des Klemmrings erfolgt, wobei zu Beginn eine Linienpressung erhalten wird. Bei weiterer Verformung des Klemmrings geht dann die Linienpressung in eine Flächenpressung über, wobei gewährleistet ist, daß an den Enden der Preßlinge eine maximale Flächenpressung erhalten wird.

Nach einer weiteren sehr vorteilhaften Ausgestaltung der Erfindung steht der Klemmring an der dem Patienten zugewandten Seite des Überbrückungsbauteils über dieses hinaus. Ein vom Klemmring gehaltenes Fixierungselement kann daher über eine Länge versteift werden, die größer ist als die Bauhöhe des Überbrückungsbauteils, das somit nicht in direkten Kontakt mit dem Körpergewebe kommt.

Die Erfindung wird nachfolgend unter Bezugnahme auf die Zeichnung näher beschrieben. Es zeigen:

Fig. 1 eine medizinische Fixierungsvorrichtung nach einem ersten Ausführungsbeispiel,

Fig. 2 die Fixierungsvorrichtung nach Fig. 1 ohne Fixierungselement und mit abgewandeltem Überbrückungsbauteil,

Fig. 3 eine medizinische Fixierungsvorrichtung nach einem zweiten Ausführungsbeispiel,

Fig. 4 die Fixierungsvorrichtung nach Fig. 3 in vergrößerter Darstellung, wobei Klemmschraube und Klemmring abgewandelt sind, und

Fig. 5 eine Draufsicht auf den Klemmring nach Fig. 4.

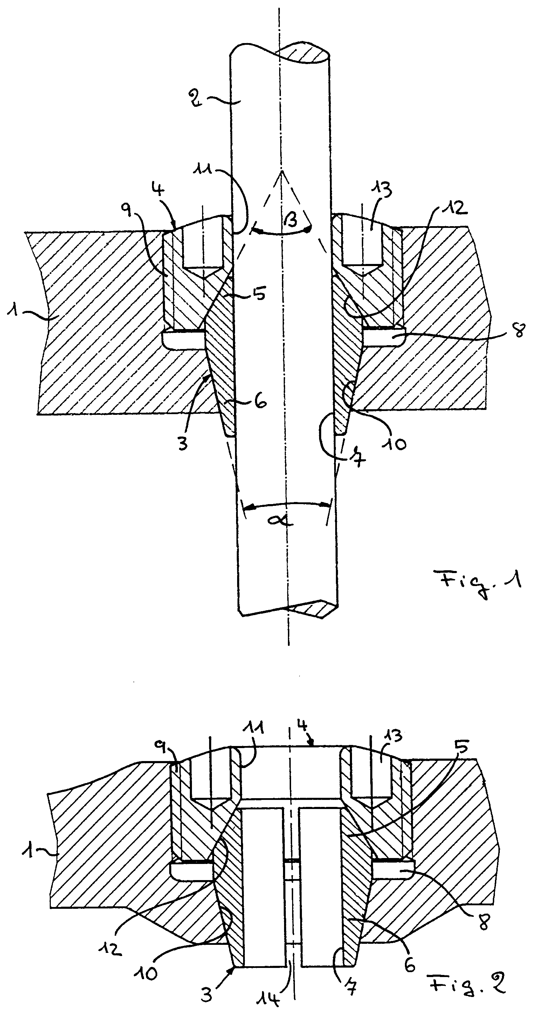

Sämtliche Figuren zeigen vergrößerte Darstellungen der medizinischen Fixierungsvorrichtung nach der Erfindung, die z. B. in der Orthopädie Verwendung finden kann. Der Maßstab in den Fig. 1 bis 3 beträgt etwa 5:1.

Ein erstes Ausführungsbeispiel der Erfindung wird nachfolgend unter Bezugnahme auf die Fig. 1 näher beschrieben. Die medizinische bzw. orthopädische Fixierungsvorrichtung enthält ein Überbrückungsbauteil 1, das aus einem langgestreckten Körper besteht, der z. B. platten- oder stabförmig ausgebildet sein kann. Der Querschnitt des Überbrückungsbauteils 1 in den verschiedenen Richtungen läßt sich in Übereinstimmung mit den gestellten Anforderungen sowie im Hinblick auf die Festigkeit des ausgewählten Materials in geeigneter Weise wählen. Ein Fixierungselement 2, beispielsweise eine Knochenschraube oder ein Knochennagel, verläuft unter einem vorbestimmten Winkel zum Überbrückungsbauteil 1, im vorliegenden Fall unter einem Winkel von 90° zu dessen Oberflächen.

Um das Fixierungselement 2 fest mit dem Überbrückungsbauteil 1 verbinden zu können, ist eine Klemmeinrichtung vorgesehen, zu der ein Klemmring 3 und eine Klemmschraube 4 gehören.

Der Klemmring 3 ist in Form eines Doppelkegels ausgebildet, weist also an gegenüberliegenden Axialseiten jeweils einen Konus 5 und 6 sowie eine koaxiale Zentralbohrung 7 auf. Der Innendurchmesser der koaxialen Zentralbohrung 7 ist so an den Außendurchmesser des Fixierungselements 2 angepaßt, daß der Klemmring 3 ohne zu großes Spiel, jedoch leicht auf das Fixierungselement 2 aufgeschoben werden kann.

Innerhalb des Überbrückungsbauteils 1 befindet sich an dessen oberer Seite eine zylindrische Ausnehmung 8, die an ihrer Umfangsfläche mit einem Innengewinde 9 versehen ist, in das die Klemmschraube 4 mit einem entsprechenden Außengewinde einschraubbar ist. Koaxial zur zylindrischen Ausnehmung 8 befindet sich in deren Bodenbereich eine Durchgangsöffnung im Überbrückungsbauteil 1, durch die eine kegelstumpfartige erste Pfanne 10 erhalten wird, deren Durchmesser sich ausgehend vom Bodenbereich der zylindrischen Ausnehmung 8 in Richtung zum Boden des Überbrückungsbauteils 1 verkleinert. Der Kegelbzw. Öffnungswinkel $\alpha$ der ersten Pfanne 10 kann im Bereich von 10° bis 40° liegen, ist also relativ spitz. Diese erste Pfanne 10 dient zur Aufnahme des Klemmrings 3 über dessen zweiten Konus 6, wobei dieser zweite Konus 6 denselben Kegelwinkel wie die erste Pfanne 10 aufweist und mit seinem Außendurchmesser an deren Innendurchmesser angepaßt ist. Der Konus 6 ruht somit in der ersten Pfanne 10.

Die Klemmschraube 4 ist mit einer zentralen Durchgangsöffnung 11 versehen, durch die das Fixierungselement 2 hindurchlaufen kann. Beispielsweise läßt sich das Fixierungselement 2 kurz oberhalb der Klemmschraube 4 abtrennen. Ferner weist die Klemmschraube 4 an ihrer dem Klemmring 3 zugewandten Stirnseite eine kegelstumpfartige zweite Pfanne 12 auf, deren Durchmesser sich ausgehend von der dem Klemmring 3 zugewandten Klemmschrauben-Stirnfläche in Richtung zum oberen Ende der Klemmschraube 4 hin verkleinert. Der Keil- bzw. Öffnungswinkel der zweiten Pfanne 12 ist mit dem Bezugszeichen $\beta$ versehen und ist größer als der Öffnungswinkel $\alpha$ der ersten Pfanne 10. Vorzugsweise liegt der Winkel $\beta$ der zweiten Pfanne 12 im Bereich zwischen 50° bis 100°. Der Winkel $\beta$ ist also wesentlich stumpfer als der Winkel $\alpha$. Der Kegelwinkel vom ersten Konus 5 des Klemmrings 3 stimmt mit dem Winkel $\beta$ im wesentlichen überein, wobei der Außendurchmesser des Konus 5 an den Innendurchmesser der zweiten Pfanne 12 angepaßt ist. Wird die Klemmschraube 4 in die zylindrische Ausnehmung 8 hineingedreht, so kommen die Flächen von erstem Konus 5 und zweiter Pfanne 12 schon aufeinander zu liegen, bevor die Klemmschraube 4 ihre Endstellung erreicht hat.

Die Klemmschraube 4 ist an ihrer Oberseite z. B. noch mit Sacklochbohrungen 13 versehen, die als Werkzeuganschluß dienen, um die Klemmschraube 4 festziehen zu können. Der Werkzeuganschluß kann je nach Zweckmäßigkeit beliebig anders gestaltet sein, z. B. als Schlitz, Innen- oder Außensechskant, usw.

Grundsätzlich kann der Klemmring 3 als ununterbrochener Ring ausgebildet sein. Er besteht aus einem Material, das sich plastisch oder elastisch verformen kann, so daß sich beim Hereindrücken des Klemmrings 3 in die kegelstumpfartige erste Pfanne 10 bei einer entsprechenden Drehbewegung der Klemmschraube 4 der Durchmesser seiner koaxialen Zentralbohrung 7 verringert, wodurch das Fixierungselement 2 eingeklemmt wird.

Der Klemmring 3 kann aber auch, wie die Fig. 2 zeigt, an einer Seite mit einem axialen Durchgangsschlitz 14 versehen sein. Hierdurch läßt er sich besonders leicht und unter gleichmäßigerer Beaufschlagung der Umfangswandung des Fixierungselements 2 verformen. Dies ist von Vorteil, wenn Fixierungselemente 2 mit unterschiedlichen Durchmessern verwendet werden.

Wie ausführlich dargelegt, erfolgt die Verklemmung des Fixierungselements 2 am Überbrückungsbauteil 1 mit Hilfe des Doppelkegel-Klemmrings 3, der unterschiedliche Kegelwinkel aufweist. Dabei ist erfindungsgemäß der Winkel $\beta$ größer als der Winkel $\alpha$. Beim Anziehen der Klemmschraube 4 wird zunächst der schlanke Kegel 6 des Klemmrings 3 infolge des kleinen Öffnungswinkels $\alpha$ bei gleichzeitiger Klemmung des Fixierungselements 2 in der ersten Pfanne 10 festgesetzt, wobei eine Sicherung des Fixierungselements 2 gegen Verdrehen und axiales Verschieben relativ zum Überbrückungsbauteil 1 zustande kommt. Bei weiterem Festziehen der Klemmschraube 4 dreht sich dann das Fixierungselement 2 nicht mehr mit, so daß nur noch die Klemmwirkung bzw. Klemmkraft vergrößert wird. Andererseits findet noch eine weitere Verklemmung der Klemmschraube 4 mit dem ersten Konus 5 des Klemmrings 3 statt. Diese Verklemmung bewirkt eine Sicherung der Klemmschraube 4 gegen Lockerung, und zwar auch dann, wenn die Fixiervorrichtung über sehr lange Zeiträume von z. B. einem Jahr oder mehr einer dynamischen bzw. rüttelnden Beanspruchung ausgesetzt ist.

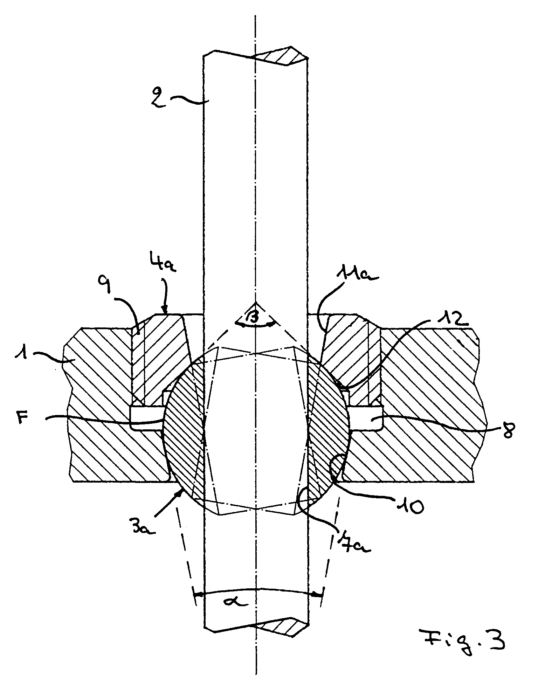

Ein zweites Ausführungsbeispiel der Erfindung ist in der Fig. 3 dargestellt. Gleiche Elemente wie in den Fig. 1 und 2 sind mit den gleichen Bezugszeichen versehen und werden nicht nochmals beschrieben.

In Abweichung von den Fig. 1 und 2 weist die Fixierungsvorrichtung nach Fig. 3 einen Klemmring 3a auf, der eine kugelförmige Außenfläche F besitzt. Der Klemmring 3a besteht im wesentlichen aus einer Kugel, die mit einer zentralen Durchgangsöffnung 7a versehen ist, die der koaxialen Zentralbohrung 7 in den Fig. 1 und 2 entspricht. Sie dient ebenfalls zur Aufnahme eines Fixierungselements 2. Der Klemmring 3a weist einen solchen Kugeldurchmesser auf, daß seine kugelförmige Außenfläche F tangential an den Flächen der ersten Pfanne 10 und der zweiten Pfanne 12 anliegt. Auch hier ist wiederum der Öffnungswinkel $\beta$ der zweiten Pfanne größer als der Öffnungswinkel $\alpha$ der ersten Pfanne 10. Um ein Verschwenken des Fixierungselements 2 relativ zum Überbrückungsbauteil 1 bei Verdrehung des Klemmrings 3a zu ermöglichen, weist die Klemmschraube 4a oberhalb der zweiten Pfanne 12 keine zylindrische, sondern eine kegelförmige Durchgangsöffnung 11a auf, deren Durchmesser sich nach außen hin vergrößert.

Die Verklemmung der Fixierungselements 2 erfolgt im vorliegenden Fall mit Hilfe des kugelförmigen Klemmrings 3a, der geschlitzt oder ungeschlitzt sein kann. Für den Fall eines ungeschlitzten Klemmrings 3a besteht dieser aus leicht verformbarem Material, das entweder elastisch oder plastisch verformbar ist. Beim Anziehen der Klemmschraube 4a wird zunächst der Klemmring 3a im schlanken Kegel des Überbrückungsbauteils 1, also in der ersten Pfanne 10, festgesetzt, wobei gleichzeitig eine Einklemmung des Fixierungselements 2 erfolgt. Er wird somit gleich zu Beginn des Einklemmvorgangs gegen Verdrehen und axiales Verschieben blockiert. Diese Blockierung kann in der zuvor beschriebenen Winkelstellung erfolgen, die sich in gewissen Bereichen durch vorhergehende Verschwenkung des Klemmrings 3a einstellen läßt. Bei weiterem Festziehen der Klemmschraube 4a wird nur noch die Klemmkraft erhöht, wobei jetzt allerdings noch die Klemmschraube 4a und der Klemmring 3a miteinander verklemmt werden, so daß die Klemmschraube 4a gegen eine unbeabsichtigte Lockerung gesichert ist.

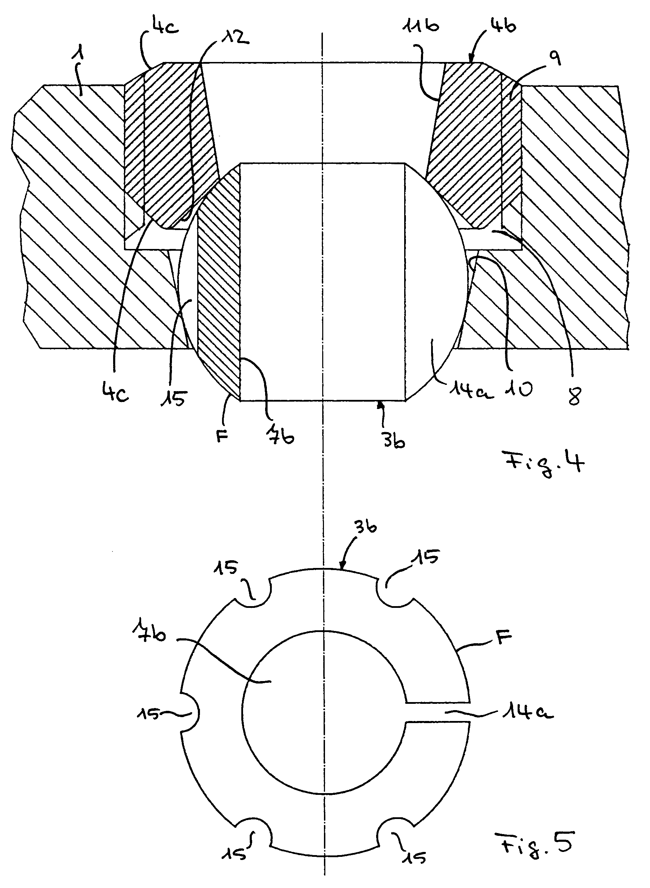

Die Fig. 4 und 5 zeigen ein weiteres Ausführungsbeispiel der Erfindung, wobei gleiche Teile wiederum mit den gleichen Bezugszeichen versehen sind und nicht nochmals beschrieben werden.

Der Klemmring 3b in den Fig. 4 und 5 entspricht im wesentlichen dem Klemmring 3a nach Fig. 3, weist aber an einer Umfangsposition zwischen seiner zentralen Durchgangsöffnung 7b und seiner Außenseite einen axialen Längsschlitz 14a auf. Beim Einklemmen des Klemmrings 3b werden also die einander zugewandten Flächen des Schlitzes 14a aufeinander zu bewegt. Hierdurch läßt sich der Klemmring 3b in möglichst gleichförmiger Weise an den Umfang des Fixierungselements 2 anlegen. Um diesen Effekt noch zu vergrößern, weist der Klemmring 3b an seiner äußeren Umfangsfläche axial verlaufende Ausnehmungen 15 auf, die dort unter im wesentlichen gleichem Abstand angeordnet sind. Durch diese Ausnehmungen 15 läßt sich die Federkonstante des Klemmrings 3b verringern, so daß der Kraftaufwand zum Einklemmen eines Fixierungselements 2 verkleinert wird. Außerdem kann durch die Ausnehmungen 15 der Klemmring 3b noch gleichförmiger gegen die Umfangsfläche des Fixierungselements 2 gepreßt werden.

Die in Fig. 5 gezeigten Ausnehmungen 15 weisen ein halbkreisförmiges Profil auf und können sich auch im Klemmring 3 von Fig. 2 befinden.

Die Klemmschraube 4b gemäß Fig. 4 besitzt wiederum eine kegelförmige Durchgangsöffnung 11b oberhalb der zweiten Pfanne 12, um eine Verkippung des Fixierungselements (nicht dargestellt) relativ zum Überbrückungsbauteil 1 zu ermöglichen, wie bereits im Zusammenhang mit der Fig. 3 beschrieben worden ist. Die Klemmschraube 4b kann eine nach außen abgeschrägte Flanke 4c besitzen, damit sie möglichst weit in die zylindrische Ausnehmung 8 hineingeschraubt werden kann, falls dies die Größe des Klemmrings 3b erfordert.

**Patentansprüche**

1. Medizinische Fixierungsvorrichtung für die Osteosynthese, bestehend aus einem langgestreckten Überbrückungsbauteil (1), mit welchem mit Knochen verbindbare stabförmige Fixierungselemente (2) über einen sie axial aufnehmenden Klemmring (3, 3a, 3b) verklemmbar sind, der in einer kegelstumpfartigen ersten Pfanne (10) des Überbrückungsbauteils (1) liegt und durch eine in das Überbrückungsbauteil (1) einschraubbare Klemmschraube (4, 4a, 4b), die eine kegelstumpfartige zweite Pfanne (12) zur Aufnahme des Klemmrings (3, 3a, 3b) aufweist, verformbar ist, **dadurch gekennzeichnet**, daß ein Öffnungswinkel ($\alpha$) der kegelstumpfartigen ersten Pfanne (10) kleiner ist als ein Öffnungswinkel ($\beta$) der kegelstumpfartigen zweiten Pfanne (12).

2. Medizinische Fixierungsvorrichtung nach Anspruch 1, **dadurch gekennzeichnet**, daß der Klemmring (3) an seinen gegenüberliegenden Enden jeweils einen konusförmigen Verlauf aufweist, der mit dem der jeweiligen ersten bzw. zweiten Pfanne (10, 12) übereinstimmt.

3. Medizinische Fixierungsvorrichtung nach Anspruch 1, **dadurch gekennzeichnet**, daß der Klemmring (3a, 3b) eine kugelförmige Außenfläche (F) aufweist, zu der die Pfannenflächen tangential verlaufen.

4. Medizinische Fixierungsvorrichtung nach Anspruch 1, 2 oder 3, **dadurch gekennzeichnet**, daß der Klemmring (3, 3a, 3b) an einer Umfangsposition vollständig in Axialrichtung geschlitzt ist.

5. Medizinische Fixierungsvorrichtung nach einem der Ansprüche 1 bis 4, **dadurch gekennzeichnet**, daß der Klemmring (3, 3a, 3b) an seiner äußeren Umfangsfläche axial verlaufende Ausnehmungen (15) aufweist.

6. Medizinische Fixierungsvorrichtung nach einem der Ansprüche 1 bis 5, **dadurch gekennzeichnet**, daß der Öffnungswinkel ($\alpha$) der kegelstumpfartigen ersten Pfanne (10) im Bereich von 10° bis 40° liegt.

7. Medizinische Fixierungsvorrichtung nach einem der Ansprüche 1 bis 5, **dadurch gekennzeichnet**, daß der Öffnungswinkel ($\beta$) der kegelstumpfartigen zweiten Pfanne (12) im Bereich von 50° bis 100° liegt.

8. Medizinische Fixierungsvorrichtung nach Anspruch 6 und 7, **dadurch gekennzeichnet**, daß der Öffnungswinkel ($\alpha$) der ersten Pfanne (10) bei etwa 24° und der ($\beta$) der zweiten Pfanne (12) bei etwa 60° liegen.

9. Medizinische Fixierungsvorrichtung nach einem der Ansprüche 1 bis 8, **dadurch gekennzeichnet**, daß die Klemmschraube (4, 4a, 4b) als Schraube mit flachem Kopf oder als solche ohne Kopf ausgebildet ist.

10. Medizinische Fixierungsvorrichtung nach einem der Ansprüche 1 bis 9, **dadurch gekennzeichnet**, daß der Öffnungswinkel ($\alpha$) der ersten Pfanne (10) bis zu 4° größer ist als der Kegelwinkel des in ihr liegenden zweiten Konus (6) des Klemmrings (3).

11. Medizinische Fixierungsvorrichtung nach einem der Ansprüche 1 bis 10, **dadurch gekennzeichnet**, daß der Öffnungswinkel ($\beta$) der zweiten Pfanne (12) bis zu 4° größer ist als der Kegelwinkel des in ihr liegenden ersten Konus (5) des Klemmrings (3).

12. Medizinische Fixierungsvorrichtung nach einem der Ansprüche 1 bis 11, **dadurch gekennzeichnet**, daß der Klemmring (3, 3a, 3b) an der dem Patienten zugewandten Seite des Überbrückungsbauteils (1) über dieses hinaussteht.

Fig. 1

Fig. 2

Fig. 3

Fig.4

Fig.5

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y,D | EP-A-0 240 034 (WITZEL) <br><br> * Seite 16, Zeile 23 - Seite 17, Zeile 34; Abbildung 14 * <br> --- | 1,3,4, 6-8, 10-12 | A61B17/60 |
| Y | US-A-4 127 119 (KRONNER) <br><br> * Spalte 7, Zeile 34 - Spalte 8, Zeile 13; Abbildung 17 * <br> --- | 1,3,4, 6-8, 10-12 | |
| A | GB-A-2 085 537 (FUJI METAL MFG.CO.LTD.) | 1,4,6-8, 10-12 | |
| A | * Seite 2, Zeile 15 - Zeile 26; Abbildungen 1,2 * <br> --- | | |
| A | FR-A-2 499 400 (ETABLISSEMENTS TORNIER ET AL.) <br> * Seite 2, Zeile 28 - Seite 3, Zeile 4; Abbildung 2 * <br><br> ----- | 1,3 | |

| | | | RECHERCHIERTE SACHGEBIETE (Int. Cl.5 ) |
|---|---|---|---|
| | | | A61B |

Der vorliegende Recherchenbericht wurde für alle Patentansprüche erstellt

| Recherchenort | Abschlußdatum der Recherche | Prüfer |
|---|---|---|
| DEN HAAG | 31 JANUAR 1992 | GIMENEZ BURGOS R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

 

& : Mitglied der gleichen Patentfamilie, übereinstimmendes Dokument

EPO FORM 1503 03.82 (P0403)